# EUROPÄISCHE PATENTSCHRIFT

(11) **EP 3 911 245 B1**
(45) Veröffentlichungstag und Bekanntmachung des Hinweises auf die Patenterteilung: **22.07.2026**
(21) Anmeldenummer: 20701155.2
(22) Anmeldetag: 14.01.2020
(51) Int. Cl.: A61B 17/00, A61B 17/16, A61B 90/90

(54) **INTEGRIERTE LEISTUNGSEINHEIT IPU**
INTEGRATED POWER UNIT IPU
UNITÉ DE PUISSANCE INTÉGRÉE IPU

(30) Priorität: 18.01.2019 DE 102019101308
(43) Veröffentlichungstag der Anmeldung: 24.11.2021
(73) Patentinhaber: Aesculap AG, 78532 Tuttlingen (DE)
(72) Erfinder: HÖGERLE, Roland-Alois, 78532 Tuttlingen (DE)
(74) Vertreter: Winter, Brandl - Partnerschaft mbB
(86) Internationale Anmeldenummer: PCT/EP2020/050807
(87) Internationale Veröffentlichungsnummer: WO 2020/148275

(56) Entgegenhaltungen:
- EP-A1- 3 070 627
- US-A1- 2016 375 273

## Beschreibung

### Technisches Gebiet

Die vorliegende Offenbarung betrifft eine Betätigungseinheit, insbesondere eine Fußschaltereinheit oder eine Handschaltereinheit für ein oder eines elektromotorisch betriebenen chirurgischen Instrument(s) vorzugsweise der minimalinvasiven Bauart.

Zum Betreiben eines Motorensystems sind bisher zumindest drei Leitungen/Kabel notwendig. Eine erste Leitung dient in diesem Fall als Energieversorgungsleitung und versorgt ausgehend von einem Netzanschluss eine Steuer- und Überwachungseinheit mit Strom. Eine zweite Leitung dient zum Verbinden der Steuer- und Überwachungseinheit mit dem an einem Handstück angebrachten Motorensystem. Diese Leitung dient sowohl als Energieversorgungsleitung als auch als Kommunikationsverbindung, um Daten und/oder Betriebsparameter zu übertragen. Eine dritte Leitung dient als Verbindung eines Fußschalters oder Handschalters mit der Steuer- und Überwachungseinheit.

Sowohl die vorstehend beschriebene zweite Leitung als auch die dritte Leitung laufen vom Patientenbereich, welcher im Folgenden auch als Sterilbereich bezeichnet ist, quer durch den Operationssaal. Davon geht eine erhebliche Gefahr aus, wie beispielsweise ein Defekt durch Überrollen des Kabels mit einem Wagen und/oder Krankenbett oder dergleichen. Des Weiteren bilden solche Leitungen eine Brücke zwischen dem Sterilbereich und einem unsterilen Bereich und stellen eine Sturzgefahr für das klinische Personal dar. Ebenso kann die Verwendung solcher Leitungen zu einer Entkopplung/Entkupplung während dem Betrieb führen. Ein weiterer Nachteil dieser Leitungen ist die notwendige Länge der jeweiligen Leitungen/Kabel. Die erste Leitung benötigt bereits eine Länge von zumindest 1,5 bis 5 m. Die zweite Leitung hat eine Länge von zumindest 4 m und die dritte Leitung misst zumindest 5 m. Abgesehen von den vorstehend beschriebenen Nachteilen für das Personal und/oder den Patienten, können die jeweiligen Kabel weitreichende technische Probleme verursachen, wie beispielsweise im Bereich der EMV (Elektromagnetische Verträglichkeit) und/oder Signalübertragung der Motorbestromung und/oder Datenübertragung zwischen Handstück und Steuergerät, usw.

Aus dem Stand der Technik sind bereits Betätigungseinheiten bekannt. Aus der DE 10 2013 114 918 A1 ist beispielsweise ein medizinischer Schalter, insbesondere ein Fußschalter, mit einer Schalterbasis und einem abnehmbaren Betätigungselement zur Betätigung des Schalters bekannt, wobei das Betätigungselement mittels zumindest eines Achselements relativ zur Schalterbasis zwischen einer ersten Schaltstellung und einer zweiten Schaltstellung positionierbar gelagert ist und ein Sicherungsmittel vorgesehen ist, das das Betätigungselement zumindest in der ersten Schaltstellung und der zweiten Schaltstellung an der Schalterbasis relativ zu dieser in eine Freigabestellung bringbar ist uns es dabei zu einer Relativpositionierung des Betätigungselements und des Sicherungsmittels kommt, so dass das Betätigungselement von der Schalterbasis abgenommen werden kann.

Aus der DE 10 2005 029 458 A1 ist eine Betätigungsvorrichtung für elektromedizinische Geräte, insbesondere Fußschalter, bekannt mit einem Bodenteil, mindestens einem Pedalteil, das mit dem Bodenteil schwenkbeweglich verbunden ist, und mindestens einem Schaltelement 12, das durch das Pedalteil betätigbar ist.

Aus der WO 01/21056 A2 ist eine chirurgische Werkzeugstation mit mehreren Werkzeugen beschreiben, wobei zu jedem Werkzeug ein eigener Fußschalter unter dem Operationstisch angeordnet ist.

In der EP 3 032 513 B1 wird ein Pairing-Verfahren zur kommunikationstechnischen drahtlosen Verbindung einer Bedieneinheit, insbesondere einer Fußschaltereinrichtung, einer medizintechnischen Steuerungseinheit, insbesondere einer Fußsteuerungseinheit, mit einem Steuergerät der medizintechnischen Steuerungseinheit beschrieben, wobei die Bedieneinheit und das Steuergerät drahtlos miteinander kommunizieren. Sie betrifft des Weiteren eine medizintechnische Steuerungseinheit, insbesondere eine Fußsteuerungseinheit mit einer Bedieneinheit, insbesondere einer Fußschaltereinrichtung, und einem mit dieser drahtlos kommunikationstechnisch verbindbaren Steuergerät, wobei die Bedieneinheit zumindest zwei Bedienelemente aufweist, bei deren jeweiliger nutzerseitiger Betätigung von dem Steuergerät ein Steuersignal an die medizintechnische Einrichtung zu deren Steuerung gegeben wird.

Die EP 1 567 063 B1 betrifft Fußschalter, die beim Betrieb mikrochirurgischer Systeme eingesetzt werden. Ein mikrochirurgisches System ist mit einem Computer und einem Fußschalter, die funktionsfähig mit dem Computer gekoppelt sind, vorgesehen.

Die EP 1 326 565 B1 bezieht sich auf ein mikrochirurgisches Steuerungssystem mit einem Computer, einem Fußschalter, der funktionsfähig mit dem Computer gekoppelt ist, und einer Touch-Bildschildschirmanzeige, die funktionsfähig mit dem Computer gekoppelt ist.

US 2016/375273 A1 beschreibt ein medizinisches Behandlungsgerät das aus einem Behandlungswerkzeug und einem Kontrollgerät besteht. Das Kontrollgerät beinhaltet eine Energiequelle und versorgt das Behandlungsinstrument mit Energie.

Somit ist es in dem Stand der Technik nachteilig, dass beispielsweise die Verwendung eines Funkfußschalters lediglich die vorstehend beschriebene dritte Leitung ersetzt, wodurch weder die Baugröße, die Kosten noch die Zuverlässigkeit optimal ist. Zudem bilden die hierbei verwendeten Kabel/Leitungen gemäß der vorstehenden beschriebenen ersten und zweiten Leitung immer noch eine Brücke vom Sterilbereich in den unsterilen Bereich.

Des Weiteren ist es nachteilig, dass die auf dem Markt erhältlichen Produkte ein Batteriepack verwenden, das aus mehreren in Reihe geschalteten Batterien besteht. Dies ist notwendig, da normalerweise jede einzelne Batterie etwa 1,3 VDC liefert. Um die Nennspannung für die verschiedenen Geräte bzw. Instrumente zu erreichen, müssen solche Einzelbatterien ihre Spannung summieren. Dieser Zustand führt zu schweren und teuren Leistungsmodulen.

Bei der Verwendung von Akkumulatoren ist die Baugröße des motorbetriebenen chirurgischen Systems inakzeptabel und es liegt eine ungenügende Performance und Zuverlässigkeit vor.

### Kurzbeschreibung der Erfindung

Der vorliegenden Erfindung liegt daher die Aufgabe zugrunde, eine Betätigungseinheit für ein chirurgisches Instrument oder eines elektromotorisch betriebenen chirurgischen Instruments bereitzustellen, welches die Nachteile aus dem Stand der Technik vermeidet oder zumindest verbessert.

Der Kern der vorliegenden Erfindung liegt darin, eine Betätigungseinheit zur Verfügung zu stellen, mit welcher ein elektromotorisch betriebenes chirurgisches Instrument betrieben werden kann, ohne dass eine Verbindung aus dem Sterilbereich in den unsterilen Bereich nötig ist. Mit anderen Worten heißt das, dass weder die Betätigungseinheit noch das elektromotorisch betriebene chirurgische Instrument über eine Leitung/ein Kabel mit einer externen Steuerungs- und/oder Überwachungseinheit, welche außerhalb des Sterilbereichs liegt, verbunden ist.

Die Aufgabe der Erfindung wird dadurch gelöst, dass eine Betätigungseinheit, insbesondere Fußschaltereinheit oder Handschaltereinheit, mit einem zu einem chirurgischen Instrument separaten sowie räumlich beabstandeten Schaltergehäuse, das mit einem Gestell, Sockeln oder dergleichen Auflagermittel zum Auflagern des Schaltergehäuses auf einem Untergrund, vorzugsweise einem Boden, oder Befestigungsmittel zum Befestigen an einem Befestigungsgrund, wie beispielsweise einem Operationstisch oder einem Roboterarm, ausgebildet oder versehen ist. Die Oberseite des Schaltergehäuses dient zugleich als Gaspedal, und wird durch Drücken der Oberseite des Schaltergehäuses in Richtung Boden/Untergrund/Befestigungsgrund betätigt. Des Weiteren ist die Betätigungseinheit mit zumindest einer Energieversorgungseinheit, vorzugsweise einer Energiespeichereinheit, ausgebildet, die für eine Energieversorgung zumindest eines Elektromotors eines an die Betätigungseinheit angeschlossenen chirurgischen Instruments ausgelegt ist. Zudem hat die Betätigungseinheit eine Steuer- und Überwachungseinrichtung, die dafür ausgebildet ist, zumindest eine der nachfolgenden Funktionen auszuführen und/oder zu überwachen.

Die Steuer- und Überwachungseinheit kann einen Energiespeichereinheit-Ladezustand überwachen anzeigen und/oder eine Energiezufuhr zu einem daran angeschlossenen chirurgischen Instrument ein-/ausschalten und/oder Daten von der Betätigungseinheit empfangen und/oder Daten an die Betätigungseinheit senden und/oder Betriebsparameter und/oder Betriebssignale an ein daran angeschlossenes chirurgisches Instrument, vorzugsweise bezüglich dessen Elektromotors, ausgeben und/oder von diesem empfangen und/oder Daten und/oder Betriebsparameter speichern.

Ferner ist die Betätigungseinrichtung mit einem Kabel und/oder einem Kabelanschluss zum Anschließen des Kabels zur elektrischen Kopplung der Betätigungseinheit mit einem chirurgischen Instrument für ein Betreiben von dessen Elektromotor mittels der in der Betätigungseinheit integrierten Energieversorgungseinheit und vorzugsweise für ein Übertragen der Daten und/oder Betriebsparameter, insbesondere sicherheitsrelevanter elektrischer Signale und Daten, ausgebildet.

In anderen Worten bedeutet das, dass die gesamte Versorgung des Motors sowie die Kommunikation zwischen der Steuer- und/oder Überwachungseinheit über genau ein Kabel läuft/stattfindet, welches die Betätigungseinheit und das elektromotorisch betriebene chirurgische Instrument miteinander verbindet. Somit wird durch die Lösung der vorliegenden Aufgabe realisiert, dass keine Kabel/Leitungen eine Brücke in den unsterilen Bereich bilden. Der gesamte Aufbau befindet sich also im Sterilbereich.

Es ist bevorzugt, wenn die Steuer- und Überwachungseinrichtung eine IPU-Einheit ist beziehungsweise als eine IPU-Einheit ausgebildet ist, mit einer Energiespeichereinheit, einer Signalgebervorrichtung, einer Motorbestromungsvorrichtung, einer Datenerfassungsvorrichtung und einer Sendeeinheit und die IPU-Einheit in dem Schaltergehäuse der Betätigungseinheit integriert ist. Mit anderen Worten heißt das, dass die gesamte Versorgung des Motors sowie neue Funktionen in der Betätigungseinheit, insbesondere dem Fußschalter oder dem Handschalter, verbaut/angebracht sind. Somit ist die Energiespeichereinheit als eine Stromversorgung in dem Schaltergehäuse integriert und kann sowohl das elektromotorisch betriebene chirurgische Instrument mit dessen Motorsystem als auch alle weiteren im Schaltergehäuse integrierten Komponenten der IPU mit Energie versorgen.

Durch den integrierten Signalgeber wird beispielsweise ein Signal ausgegeben, welches beispielsweise einen Rechtslauf des Elektromotors vorgibt. Genauso kann die Motorbestromung gesteuert werden und gegebenenfalls auch automatisch an das jeweilige an das Motorsystem angeschlossene Anwendungsteil/Handstück angepasst werden. Die in dem Betätigungsteil eingebaute Datenerfassung dient beispielsweise zum Erfassen von Aufbereitungszyklen, einer Aufbereitungsdauer, Waschzyklen und/oder dem Pflegestatus des Motors. Die Sendeeinheit sendet Daten und/oder Betriebsparameter an eine externe Einheit, wie ein Smartphone oder eine Ladestation.

Darüber hinaus ist es bevorzugt, wenn in der Motorbestromungsvorrichtung ein DC/DC-Wandler verwendet ist, der zusätzlich in der Betätigungseinheit integriert ist, um die von der Energiespeichereinheit gelieferte Spannung von 12 VDC auf 36 VDC zu erhöhen, was die Spannung ist, die zum Betreiben eines Umrichter-gespeisten Permanentmagnetsynchronmotors benötigt wird.

Hierbei ist es bevorzugt, wenn der Einsatz eines Boost-Wandlers zwei der drei bisher benötigten Akkupacks ersetzt, um ein Operationsgerät zu betreiben, das beispielsweise eine Eingangsspannung von 36 VDC benötigt. Dies hat den Vorteil eines erheblichen Kostenersparnisses, da die grobe Berechnung an Ionen zeigt, dass die Kosten für einen Serien-Boost-Wandler deutlich, insbesondere 10-mal geringer sind als die Kosten für zwei Akkupacks.

Des Weiteren ist es bevorzugt, dass eine freie Einstellung der Spannungsverstärkung den Einsatz beliebiger Batterietechnologie ermöglicht wird. Dies bietet den Vorteil, dass durch diese Flexibilität die Abhängigkeit von einzelnen Anbietern oder möglichen Auslaufprodukten entfällt. Zudem ist das Gewicht des Umrichters wesentlich geringer als das Gewicht der Batterien. Daraus resultiert eine Verbesserung in der Handhabung des Geräts. Ein niedriges Gesamtgewicht ist insbesondere bei Geräten, die vom Operateur direkt gehalten werden müssen besonders wichtig.

Darüber hinaus ermöglicht die Erfindung bei einer bestimmten Größe und einem bestimmten Volumen den Betrieb des Motors mit einem deutlich höheren Spannungsniveau. Dies führt zu einem deutlich niedrigeren Stromniveau, einem höheren Wirkungsgrad und niedrigeren Kosten der Motorenproduktion.

Des Weiteren sind Antriebssysteme in Operationssälen für beispielsweise die Knochenbehandlung entweder für batteriebetriebene Motoren entsprechend einer 12 V Versorgung oder für kabelgebundene Systeme entsprechend einer 36 V Versorgung. Die Erfindung bietet den Vorteil, dass die beiden Motorplattformen auf eine Plattform reduziert werden kann.

Durch die Reduzierung des Akkus bzw, der Energiespeichereinheit ist die Ladezeit entsprechend verkürzt. Es ist wichtig zu erwähnen, dass, obwohl die gespeicherte Energie auf ein Drittel der ursprünglichen Kapazität reduziert wird, die verfügbare Ladung problemlos einen normalen chirurgischen Eingriff überstehen sollte.

Ferner ist es bevorzugt, wenn eine weitere Variante der Erfindung in einem Elektrowerkzeug untergebracht werden. Um den Permanentmagnetsynchronmotor anzutreiben, wird ein Inverter/DC-Bus mit 36 VDC verwendet. Diese Lösung würde das Handling dieser Instrumente drastisch erhöhen.

Aufgrund wichtiger und eventuell fluidempfindlicher in dem Schaltergehäuse der Betätigungseinheit integrierter Komponenten ist es vorteilhaft, wenn das Schaltergehäuse fluiddicht ist beziehungsweise fluiddicht verschließbar ist.

Ein weiterer Vorteil der vorstehenden Ausführungsform ist die deutliche Reduzierung des Platzbedarfs in einem Operationssaal durch den kompletten/vollständigen Wegfall der Steuer- und/oder Überwachungseinheit als ein externes Gerät. Auch durch die Verwendung von nur noch einem Kabel, welches vorzugsweise die halbe Gesamtkabellänge der Leitungen aus dem vorstehend beschriebenen Stand der Technik aufweist, kann ein Vorteil erzielt werden. Durch die reduzierte Kabellänge kann eine sicherere und weniger anfällige Übertragung sicherheitsrelevanter elektrischer Signale und Daten beziehungsweise Betriebsparameter gewährleistet werden.

Ferner ist es bevorzugt, wenn die Energiespeichereinheit ein Akkumulator/Akku oder eine Batterie ist. Mehr bevorzugt ist die Verwendung eines Akkumulators, welcher wieder aufladbar ist und somit sowohl weniger kostenintensiv als auch umweltfreundlicher ist als eine Batterie. Ein weiterer Vorteil eines integrierten Akkumulators ist, dass das Kabel/die Leitung zum Versorgen der Energiespeichereinheit eingespart werden kann. Somit wird ermöglicht, dass das Gesamtsystem in einem Sterilbereich betrieben werden kann, ohne dass ein Energieversorgungskabel eine Brücke zwischen dem Sterilbereich und dem unsterilen Bereich durchbrechen muss. Durch solch eine Ausführungsform kann die Energiespeichereinheit in Abwesenheit eines Patienten, also in einem Offline-Betriebsmodus ausgetauscht beziehungsweise aufgeladen werden.

Es ist weiter bevorzugt, wenn die IPU-Einheit ausgebildet ist, um in der Betätigungseinheit, insbesondere der Fußschaltereinheit oder Handschaltereinheit, zentral Daten und/oder Betriebsparameter zu erfassen. In anderen Worten, ist in der Fußschaltereinheit oder Handschaltereinheit eine zentrale Datenerfassungseinrichtung vorgesehen, welche die Daten empfängt, speichert und wenn nötig ausgeben kann. Somit kann auf die Daten sowohl im Betriebsmodus als auch im Offline-Betriebsmodus zugegriffen werden. Des Weiteren werden durch eine solche zentrale Datenerfassung alle in einem mehr oder weniger ausgedehnten Bereich gewonnenen Daten an einem zentralen Ort zusammengeführt. Auf diese Weise können auf verschieden Kommunikations- oder Versandwege zwischen mehreren Datenerfassungseinrichtungen verzichtet werden.

Es ist weiter bevorzugt, wenn die IPU-Einheit eine Datenschnittstelle, insbesondere eine Schnittstelle zum Anstecken eines Speichermediums, zum Auslesen aller Daten und/oder Betriebsparameter, vorzugsweise der Motorenhistorie aller Anwendungsteile/Handstücke, hat. Somit bietet der vorliegende Erfindungsgegenstand die Möglichkeit, in einem Offline-Betriebsmodus auf die gespeicherten und/oder erfassten Daten und/oder Betriebsparameter der IPU-Einheit durch Anstecken eines Speichermedium an die IPU-Einheit zuzugreifen, um diese anschließend beispielsweise an einem separaten, vorzugsweise von der IPU-Einheit unabhängigen Vorrichtung, wie beispielsweise einem PC/Computer auszulesen, zu bearbeiten oder weiterzuverwenden. Ein solches Speichermedium kann beispielsweise eine USB-Stick, eine Speicherkarte oder dergleichen sein.

Ferner ist es bevorzugt, wenn die IPU-Einheit aus dem Schaltergehäuse vollständig entnehmbar/lösbar ist, insbesondere zur Reinigung und/oder Aufbereitung des Schaltergehäuses und anschließend wieder einsetzbar ist. Neben einer Reinigung und/oder Aufbereitung, vor allem der äußeren Flächen der Betätigungseinheit, ist es auch denkbar, die IPU-Einheit an beispielsweise einen Computer anzustecken, um auf die darauf gespeicherten/erfassten Daten und/oder Betriebsparameter zugreifen zu können. Des Weiteren besteht auf diese Weise die Möglichkeit, notwendige Updates und/oder neue Programme aufzuspielen/zu installieren. Ein weiterer Vorteil ist, dass das Schaltergehäuse einer intensiveren Reinigung unterzogen werden kann, wenn sich die IPU-Einheit zu diesem Zeitpunkt nicht in dem Schaltgehäuse befindet.

Es ist weiter bevorzugt, wenn die Unterseite des Schaltergehäuses mit einer rutschfesten Beschichtung versehen ist. Dies bringt den Vorteil mit sich, dass die Betätigungseinheit, in dem Fall, wenn sie von einem Bediener, vorzugsweise Klinikpersonal, insbesondere einem Operateur, betätigt wird, nicht verrutscht. Dadurch kann zudem verhindert werden, dass beispielsweise die aufzubringende Druckkraft des Bedieners variiert, wie es der Fall wäre, wenn das Schaltergehäuse beispielsweise nicht rutschfest auf dem Untergrund, vorzugsweise Boden, aufliegt. Des Weiteren ist es vorteilhaft, wenn das Betätigungselement auch bei einer unbeabsichtigten Berührung, beispielsweise einem Stoß von der Seite, seine Position nicht verändert. Trotzdem ist es vorgesehen, dass die Betätigungseinheit eine mobile und immer wieder beliebig positionierbare Einheit bleibt und nicht fest mit dem Untergrund/Boden verbunden ist.

Es ist weiter bevorzugt, wenn zwischen der IPU-Einheit und einem Ladegerät während eines Ladeprozesses ein bidirektionaler Datenaustausch vorgesehen ist und ein Datenaustausch, insbesondere aller Daten und/oder Betriebsparameter aller Anwendungsteile zwischen dem Ladegerät und einer Cloud vorgesehen ist. Durch das Speichern aller oder auch nur einiger Daten und/oder Betriebsparameter in einer Cloud besteht die Möglichkeit diese Daten auch außerhalb und in Abwesenheit der IPU-Einheit abzurufen. Ein bidirektionaler Datenaustausch bedeutet hierbei, dass eine Datenübertragung in beide Richtungen zwischen zwei Einrichtungen stattfindet. Das heißt, dass beide Einrichtungen in der Lage sind sowohl Daten zu empfangen (Receiver) als auch Daten zu senden (Transmitter). Um diese gewährleisten zu können, versteht sich von selbst, dass auch die entsprechenden Schnittstellen bidirektional ausgebildet sind.

Die Erfindung betrifft ferner eine Kommunikation der Betätigungseinheit mit einem Smartphone oder Tablet vorgesehen ist, insbesondere zur Bedienung des jeweiligen Anwendungsteils und zur Nutzung als Display der IPU-Einheit. Dadurch kann über das Smartphone oder Tablet der Ladezustand der integrierten Energiespeichereinheit kontrolliert werden und/oder die Stromzufuhr ein- oder ausgeschalten werden, also die Betätigungseinheit in Betrieb nehmen oder in den Offline-Betriebsmodus zu wechseln. Des Weiteren ist es auf diese Weise möglich verschiedene Einstellungen, wie beispielsweise das Einstellen individueller Motorparameter, oder eine individuelle Bedienung der Anwendungsteile zu nutzen. Die Kommunikationsverbindung von dem Smartphone oder Tablet zu der Betätigungseinheit erfolgt kabellos, vorzugsweise per Wifi. Alternativ besteht die Möglichkeit einer Datenübertragung per Bluetooth.

Des Weiteren betrifft die vorliegende Erfindung ein chirurgisches Behandlungssystem mit einem elektromotorisch betriebenen, chirurgischen Instrument, dessen Elektromotor, der für einen Betrieb mit einer Energiespeichereinheit ausgelegt ist, in einem Handstück aufgenommen ist, an dessen proximalen Ende oder Endbereich ein Kabelanschluss für die Energieversorgung zumindest des Elektromotors und/oder für die Übertragung von Daten und/oder Betriebsparameter ausgebildet oder angeordnet ist und einer Betätigungseinheit mit den Merkmalen gemäß einem der vorstehenden Aspekte.

Somit bietet der vorstehend beschriebene Erfindungsgegenstand sowie das chirurgische Behandlungssystem eine deutliche Platzeinsparung im Operationssaal sowie eine Kostenreduzierung im Vergleich zu bereits bekannten Aufbauten. Des Weiteren ist die Betätigungseinheit eine kompakte, leichte und zudem mobile Einheit.

### Kurzbeschreibung der Figuren

Fig. 1 ist eine Darstellung zur Veranschaulichung der Betätigungseinheit gemäß der vorliegenden Offenbarung;
Fig. 2 ist eine Darstellung zur Veranschaulichung der Steuer- und Überwachungseinheit gemäß der vorliegenden Offenbarung;
Fig. 3 ist eine Darstellung zur Veranschaulichung eines Behandlungssystems gemäß der vorliegenden Offenbarung;
Fig. 4 ist eine Darstellung zur Veranschaulichung des Behandlungssystems gemäß dem Stand der Technik; und
Fig. 5 ist eine Darstellung der Energieversorgungskomponenten des Motorensystems.

### Beschreibung der Ausführungsbeispiele

Nachstehend werden Ausführungsbeispiele der vorliegenden Offenbarung auf der Basis der zugehörigen Figuren beschrieben. Die Figuren sind lediglich schematischer Natur und dienen dem Verständnis der Erfindung. Die gleichen Elemente sind mit denselben Bezugszeichen bezeichnet.

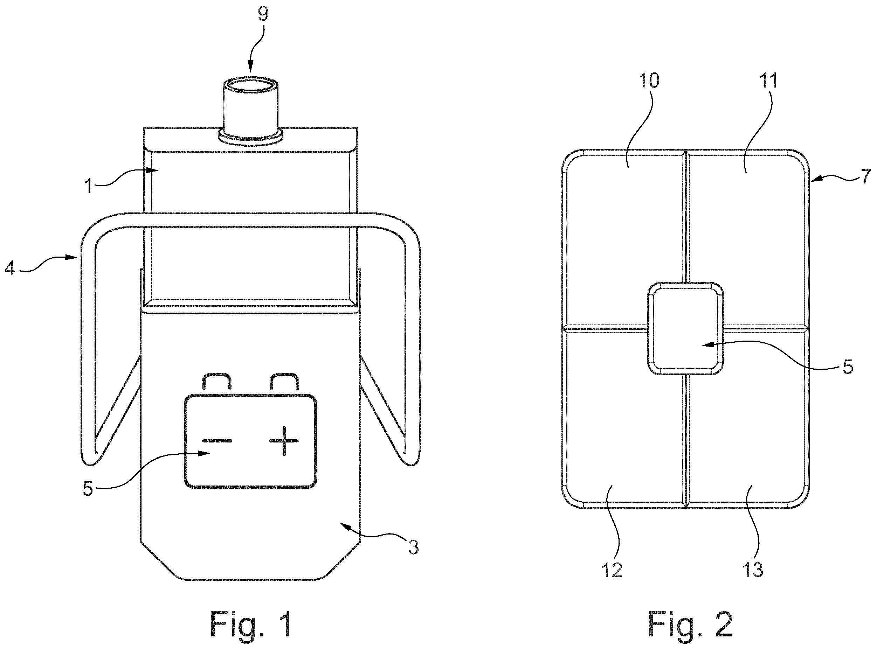

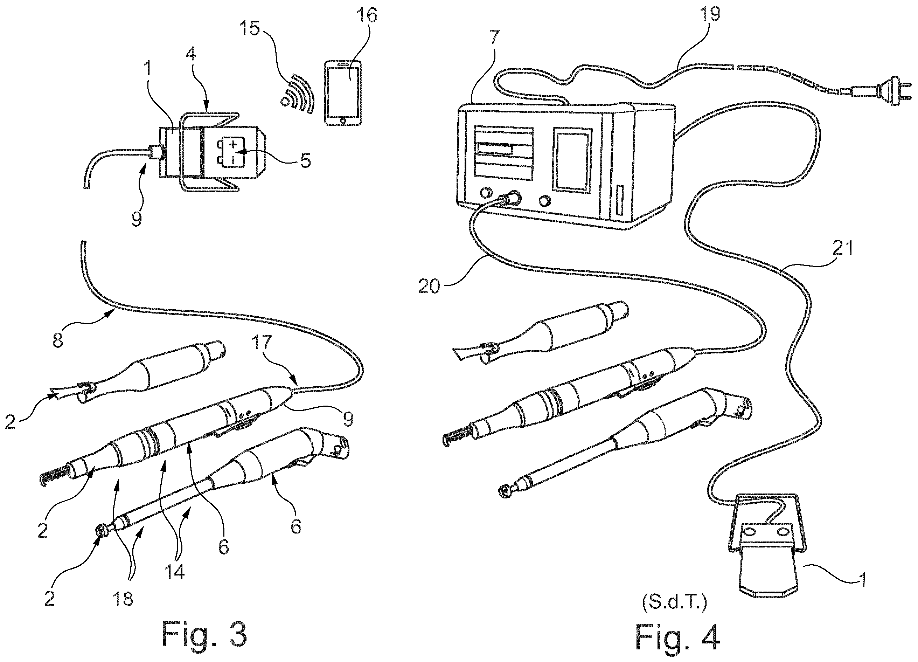

Fig. 1 ist eine Darstellung zur Veranschaulichung der Betätigungseinheit 1 gemäß der vorliegenden Offenbarung. Die Betätigungseinheit 1 ist in Fig. 1 gemäß einer bevorzugten Ausführungsform als Fußschaltereinheit ausgebildet, um mit einem elektromotorisch betriebenen chirurgischen Instrument 2 (siehe Fig. 3) in Kontakt zu stehen/kommen. Eine Handschaltereinheit ist entsprechend ähnlich ausgebildet. Die Betätigungseinheit 1 hat ein Schaltergehäuse 3, das mit einem Gestell 4 ausgebildet ist. Das Gestell 4, welches als ein feststehender Bügel über dem Schaltergehäuse 3 ausgebildet ist und auf jeweils zwei sich gegenüberliegenden Seiten an das Schaltergehäuse 3 fixiert/angebracht ist, wird zum Anheben und Verschieben der Betätigungseinheit 1 durch einen Bediener, vorzugsweise mit dessen Fuß/Hand verwendet. Die Oberseite des Schaltergehäuses 3 der Betätigungseinheit 1 wird bei Betätigung in Richtung Boden/Auflagefläche gedrückt. Die Betätigungseinheit 1 ist mit einer Energieversorgungseinheit 5 versehen, welche ein damit verbundenes Motorensystem 6 (siehe Fig. 3) mit Strom/Energie versorgt. Darüber hinaus ist zum Anbringen eines Kabels 8 (siehe Fig. 3) auf einer Seite der Betätigungseinheit 1 ein Kabelanschluss vorgesehen.

Fig. 2 ist eine Darstellung zur Veranschaulichung der Steuer- und Überwachungseinheit 7 gemäß der vorliegenden Offenbarung. Die Steuer- und Überwachungseinheit 7 ist derart vorgesehen, um in das Schaltergehäuse 3 gemäß Fig. 1 integriert zu werden. Die Steuer- und Überwachungseinheit 7 ist eine vorzugsweise komplett entnehmbare IPU-Einheit 7, bestehend aus mehreren Teilkomponenten. In Fig. 2 ist zu erkennen, dass die IPU-Einheit mit einer Signalgebereinrichtung 10, einer Motorbestromungseinrichtung 11, einer Datenerfassungseinrichtung 12 und einer Sendeeinheit 13 ausgebildet ist. Zudem hat die IPU-Einheit eine Energiespeichereinheit 5, vorzugsweise als ein Akkumulator ausgebildet, welche vorzugsweise zentral in der IPU-Einheit 7 vorgesehen ist und die umliegenden Teilkomponenten sowie das Motorensystem 6 zum elektromotorischen Betreiben des chirurgischen Instruments 2 mit Energie/Strom versorgt. Es ist bevorzugt, wenn die IPU-Einheit 7 nahezu die gleiche Form aufweist, wie das Schaltergehäuse 3 der Betätigungseinheit 1.

Fig. 3 ist eine Darstellung zur Veranschaulichung eines Behandlungssystems gemäß der vorliegenden Offenbarung. Das Behandlungssystem hat die in Fig. 1 bereits beschriebene Betätigungseinheit 1, in welche die IPU-Einheit 7 mit der Energiespeichereinheit 5 integriert ist. Über ein Kabel 8 ist das Motorensystem 6 an dessen proximalen Ende 17 mit dem Kabelanschluss 9 der Betätigungseinheit 1 verbunden. Das Motorensystem 6 ist an dessen distalen Ende 18 mit dem chirurgischen Instrument 2 verbunden. Das Motorensystem 6 und das daran angeschlossene chirurgische Instrument 2 bilden zusammen das Handstück/Anwendungsteil 14 aus.

Darüber hinaus ist es vorgesehen, dass die IPU-Einheit 7 kabellos über eine Kommunikationsverbindung 15 mit einem Smartphone 16 in Verbindung steht. Die Kommunikationsverbindung 15 ist derart ausgelegt, um beispielsweise Einstellungen individueller Motorparameter vornehmen zu können. Des Weiteren ist es bevorzugt, wenn das Display des Smartphones 16 als Display der IPU-Einheit 7 genutzt wird. Somit kann über das Smartphone 16 beispielsweise der Energiespeichereinheit-Ladezustand abgelesen und/oder die Energiezufuhr ein- und ausgeschalten werden.

Fig. 4 ist eine Darstellung zur Veranschaulichung des Behandlungssystems gemäß dem Stand der Technik. Fig. 4 entspricht im Wesentlichen der Darstellung aus Fig. 3 mit dem Unterschied, dass im Stand der Technik drei Kabel verwendet werden müssen, um alle erforderlichen und/oder gewünschten Funktionen zum Betreiben des chirurgischen Instruments 2 zu erfüllen. Somit ist in Fig. 4 einer erste Leitung 19 zu erkennen, welche eine Versorgungsleitung zwischen einem Netzteil oder einer Netz- (nicht dargestellt) und einer Steuer- und Überwachungseinrichtung 7 ist. Die zweite Leitung 20 verbindet die Steuer- und Überwachungseinrichtung 7 mit dem in das Anwendungsteil 14 integrierten Motorensystem 6, welches mit dem chirurgischen Instrument 2 verbunden ist. Die dritte Leitung 21 dient zum Verbinden der Steuer- und Überwachungsvorrichtung 7 mit der Betätigungseinheit 1.

Ein Vergleich der beiden Fign. 3 und 4 verdeutlicht, dass das eine Kabel 8 gemäß Fig. 3 sowohl die erste Leitung 19, zweite Leitung 20 als auch die dritte Leitung 21 ersetzt. Dadurch kann auf Kabel/Leitungen, welche quer durch den Operationssaal verlaufen, verzichtet werden.

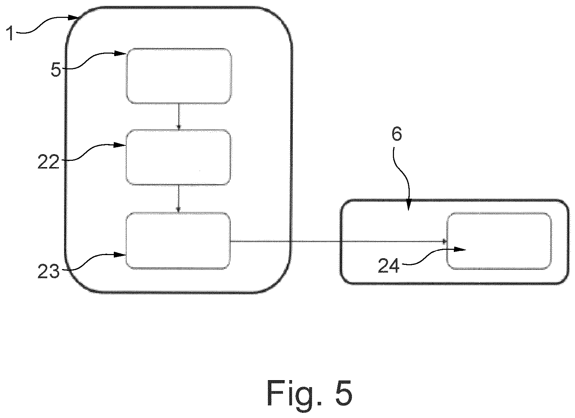

Fig. 5 ist eine Darstellung der Energieversorgungskomponenten des Motorensystems. Fig. 5 zeigt die Energieversorgungseinheit 5, einen Umrichter 22 und einen Inverter 23, welche in der Betätigungseinheit 1 integriert sind. Hierbei ist es bevorzugt, wenn der Umrichter 22 und der Inverter 23 ein Teil der Motorbestromungsvorrichtung 11 ist. Die Energiespeichereinheit 5 gibt eine 12 V Gleichspannung an den Umrichter 22, welcher die 12 V Gleichspannung in 36 V Gleichspannung umwandelt. Die 36 V Gleichspannung von dem Umrichter wird an den Inverter 23 ausgeben, welcher diese in eine Wechselspannung umwandelt und an das Motorensystem 6 ausgibt, um den darin integrierten Permanentmagnetsynchronmotor 24 mit Energie zu versorgen bzw. anzutreiben.

### Bezugszeichenliste

- 1: Betätigungseinheit
- 2: Instrument
- 3: Schaltergehäuse und Gaspedal
- 4: Gestell
- 5: Energieversorgungseinheit
- 6: Motorensystem
- 7: Steuer- und Überwachungsvorrichtung/IPU-Einheit
- 8: Kabel
- 9: Kabelanschluss
- 10: Signalgebervorrichtung
- 11: Motorbestromungsvorrichtung
- 12: Datenerfassungsvorrichtung
- 13: Sendeeinheit
- 14: Anwendungsteil
- 15: Kommunikationsverbindung
- 16: Smartphone
- 17: proximales Ende
- 18: distales Ende
- 19: erste Leitung
- 20: zweite Leitung
- 21: dritte Leitung
- 22: Umrichter
- 23: Inverter
- 24: Permanentmagnetsynchronmotor

## Patentansprüche

1. Betätigungseinheit (1), insbesondere Fußschaltereinheit oder Handschaltereinheit, für ein oder eines elektromotorisch betriebenen chirurgischen Instrument(s) (2) vorzugsweise der minimalinvasiven Bauart mit
einem zum chirurgischen Instrument (2) separaten sowie räumlich beabstandeten Schaltergehäuse (3), das mit einem Gestell (4), Sockeln oder dergleichen Auflagermittel zum Auflagern des Schaltergehäuses (3) auf einem Untergrund, vorzugsweise einem Boden, oder Befestigungsmittel zum Befestigen an einem Befestigungsgrund, vorzugsweise einem Operationstisch oder einem Roboterarm ausgebildet oder versehen ist,
zumindest einer Energieversorgungseinheit (5), vorzugsweise einer Energiespeichereinheit, die für eine Energieversorgung zumindest eines Motorensystems (6), insbesondere eines Elektromotors, des an die Betätigungseinheit (1) anzuschliessenden chirurgischen Instruments (2) ausgelegt ist,
einer Steuer- und Überwachungseinrichtung (7) die dafür ausgebildet ist, zumindest eine der nachfolgenden Funktionen auszuführen und/oder zu überwachen:
Empfangen und/oder Senden von Daten an eine externe Instrumenten-Betätigungseinheit (1), vorzugsweise einer Fußschaltereinheit oder einer Handschaltereinheit,
Ausgeben und/oder Empfangen von Betriebsparametern und Betriebssignalen an/von einem daran angeschlossenen chirurgischen Instrument (2) vorzugsweise bezüglich dessen Motorensystem, und
Speichern von Daten und/oder Betriebsparametern,
ein Kabel (8) und/oder ein Kabelanschluss (9) zum Anschließen des Kabels (8) zur elektrischen Kopplung der Betätigungseinheit (1) direkt mit einem chirurgischen Instrument (2) für ein Betreiben von dessen Motorensystem, insbesondere dessen Elektromotor, mittels der in der Betätigungseinheit (1) integrierten Energieversorgungseinheit (5) und vorzugsweise für ein Übertragen der Daten und/oder Betriebsparameter, insbesondere sicherheitsrelevanter elektrischer Signale und Daten vorgesehen ist,
wobei die Steuer- und Überwachungseinrichtung (7) im Schaltergehäuse (3) integriert ist, und es eine IPU-Einheit ist, und dass die Energieversorgungseinheit (5) in der IPU-Einheit integriert ist, und das elektromotorisch betriebene chirurgische Instrument (2) mit dessen Motorsystem (6) als auch alle weiteren im Schaltergehäuse (3) integrierten Komponenten der Steuer- und Überwachungseinrichtung (7) mit Energie versorgen kann, **dadurch gekennzeichnet, dass** eine Kommunikationsverbindung der IPU-Einheit mit einem Smartphone oder Tablet zur Bedienung des jeweiligen Anwendungsteils und zur Nutzung als Display der IPU-Einheit so vorgesehen ist, dass die IPU-Einheit den Energiespeichereinheit-Ladezustand überwachen kann, und/oder die Energiezufuhr ein- und ausschalten kann und/oder den individuellen Motorparameter einstellen kann.

2. Betätigungseinheit (1) gemäß Anspruch 1, **dadurch gekennzeichnet, dass** die Steuer- und Überwachungseinrichtung (7) die folgenden Komponenten aufweist: eine Signalgebervorrichtung (10), die derart ausgebildet ist, dass sie die Betriebsparameter und/oder die Betriebssignale an dessen Elektromotors ausgibt, eine Motorbestromungsvorrichtung (11), die derart ausgebildet ist, dass sie Motorbestromung steuert, eine zentrale Datenerfassungsvorrichtung (12), die derart ausgebildet ist, dass sie die Daten und/oder die Betriebsparameter von dem Anwendungsteil, welches das Motorensystem (6) und das daran angeschlossene
chirurgische Instrument (2) zusammen ausbilden, empfängt, speichert, erfasst und ausgibt, und eine Sendeeinheit (13), die derart ausgebildet ist, dass sie die Daten und/oder die Betriebsparameter an ein externes Smartphone oder Tablet sendet, und die IPU-Einheit (7), die in dem Schaltergehäuse (3) der Betätigungseinheit (1) integriert ist.

3. Betätigungseinheit (1) gemäß Anspruch 1, **dadurch gekennzeichnet, dass** die Energiespeichereinheit (5) zentral in der IPU-Einheit (7) vorgesehen ist.

4. Betätigungseinheit (1) gemäß einem der Ansprüche 1 bis 3, **dadurch gekennzeichnet, dass** die Energiespeichereinheit (5) ein Akkumulator oder eine Batterie ist.

5. Betätigungseinheit (1) gemäß einem der Ansprüche 2 bis 4, **dadurch gekennzeichnet, dass** die IPU-Einheit (7) ausgebildet ist, um in dem Schaltergehäuse (3) der Betätigungseinheit (1) zentral Daten und/oder Betriebsparameter zu erfassen.

6. Betätigungseinheit (1) gemäß einem der Ansprüche 2 bis 5, **dadurch gekennzeichnet, dass** die IPU-Einheit (7) eine Datenschnittstelle, insbesondere eine Schnittstelle zum Anstecken eines Speichermediums, zum Auslesen aller Daten und/oder Betriebsparameter der Motorenhistorie aller Anwendungsteile (14) hat.

7. Betätigungseinheit (1) gemäß einem der Ansprüche 2 bis 6, **dadurch gekennzeichnet, dass** die IPU-Einheit (7) aus dem Schaltergehäuse (3) vollständig entnehmbar ist, insbesondere zur Reinigung und/oder Aufbereitung des Schaltergehäuses (3).

8. Betätigungseinheit (1) gemäß Anspruch 7, **dadurch gekennzeichnet, dass** die entnommene IPU-Einheit (7) derart ausgebildet ist, um an einen Computer angesteckt zu werden, um auf die darauf gespeicherten und/oder erfassten Daten und/oder Betriebsparameter zuzugreifen.

9. Betätigungseinheit (1) gemäß Anspruch 7 oder 8, **dadurch gekennzeichnet, dass** die entnommene IPU-Einheit (7) derart ausgebildet ist, um notwendige Updates und/oder neue Programme aufzuspielen/zu installieren.

10. Betätigungseinheit (1) gemäß einem der Ansprüche 2 bis 9, **dadurch gekennzeichnet, dass** das die Unterseite des Schaltergehäuses (3) mit einer rutschfesten Beschichtung versehen ist.

11. Betätigungseinheit (1) gemäß einem der Ansprüche 2 bis 9, **dadurch gekennzeichnet, dass** zwischen der IPU-Einheit (7) und einem Ladegerät während eines Ladeprozesses ein bidirektionaler Datenaustausch vorgesehen ist und ein Datenaustausch, insbesondere aller Daten und/oder Betriebsparameter aller Anwendungsteile (14) zwischen dem Ladegerät und einer Cloud vorgesehen ist.

12. Betätigungseinheit gemäß einem der Ansprüche 2 bis 11, **dadurch gekennzeichnet, dass** eine Kommunikationsverbindung (15) der Betätigungseinheit mit einem Smartphone (16) vorgesehen ist, insbesondere zur Bedienung des jeweiligen Anwendungsteils (14) und zur Nutzung als Display der IPU-Einheit (7).

13. Chirurgisches Behandlungssystem mit einer Betätigungseinheit (1) mit den Merkmalen gemäß einem der vorstehenden Ansprüche und einem elektromotorisch betriebenen chirurgischen Instrument (2), dessen Motorensystem (6), insbesondere Elektromotor, der für einen Betrieb mit einer Energiespeichereinheit (5) ausgelegt ist, in einem Anwendungsteil (14) aufgenommen ist, an dessen proximalen Ende (17) oder Endbereich ein Kabelanschluss für die Energieversorgung zumindest des Motorensystems, insbesondere des Elektromotors, und/oder für die Übertragung von Daten und/oder Betriebsparameter ausgebildet oder angeordnet ist.

## Claims

1. An actuating unit (1), in particular a foot-operated switch unit or hand-operated switch unit, for an or of an electromotively-operated surgical instrument (2), preferably of the minimally invasive type, comprising
a switch housing (3) which is separate from and spatially spaced apart from the surgical instrument (2) and which is configured or provided with a frame (4), bases or similar supporting means for supporting the switch housing (3) on a surface, preferably a bottom, or fastening means for fastening to a fastening base, preferably an operating table or a robot arm,
at least one power supply unit (5), preferably an energy storage unit, which is configured for a power supply of at least one motor system (6), in particular of an electric motor, of the surgical instrument (2) to be connected to the actuating unit (1),
a control and monitoring device (7) configured to perform and/or monitor at least one of the following functions:
receiving and/or sending data to an external instrument-actuating unit (1), preferably a foot-operated switch unit or a hand-operated switch unit,
outputting and/or receiving operating parameters and operating signals to/from a surgical instrument (2) connected thereto, preferably with respect to its motor system, and
storing data and/or operating parameters,
a cable (8) and/or a cable connection (9) is provided for connecting the cable (8) for electrically coupling the actuating unit (1) directly to a surgical instrument (2) for operating its motor system, in particular its electric motor, via the power supply unit (5) integrated in the actuating unit (1) and preferably for transmitting the data and/or operating parameters, in particular safety-relevant electrical signals and data,
wherein the control and monitoring device (7) is integrated into the switch housing (3), and is an IPU unit, and in that the energy supply unit (5) is integrated in the IPU unit and can supply both the electromotively-operated surgical instrument (2) with its motor system (6) and components of the control and monitoring device (7) integrated in the switch housing (3) with energy, **characterized in that** a communication connection of the IPU unit with a smartphone or tablet is provided for operating the respective application part and for use as a display of the IPU unit, so that the IPU unit can monitor the state of charge of the energy or storage unit, and/or to switch the energy supply on and off and/or to set individual motor parameters.

2. The actuating unit (1) according to claim 1, **characterized in that** the control and monitoring device (7) comprises the following components: a signal-generator device (10), which is configured to output the operating parameters and/or the operating signals to the electric motor, a motor energization device (11), which is configured to control the motor system current, a central data acquisition device (12) which is configured to receive, store, acquire, and output the data and/or operating parameters of the application part which includes a motor system and a surgical instrument (2) connected to it together, and a sending unit (13), which is configured to send the data and/or operating parameters to an external smartphone or tablet, and the IPU unit (7) is integrated in the switch housing (3) of the actuating unit (1).

3. The actuating unit (1) according to claim 1, **characterized in that** the energy storage unit (5) is centrally provided in the IPU unit (7).

4. The actuating unit (1) according to one of claims 1 to 3, **characterized in that** the energy storage unit (5) is an accumulator or a battery.

5. The actuating unit (1) according to one of claims 2 or 4, **characterized in that** the IPU unit (7) is configured to centrally acquire data and/or operating parameters in the switch housing (3) of the actuating unit (1).

6. The actuating unit (1) according to one of claims 2 to 5, **characterized in that** the IPU unit (7) has a data interface, in particular an interface for plugging in a storage medium, for reading all data and/or operating parameters of the motor history of all application parts (14).

7. The actuating unit (1) according to one of claims 2 to 6, **characterized in that** the IPU unit (7) is completely removable from the switch housing (3), in particular for cleaning and/or reprocessing the switch housing (3).

8. The actuating unit (1) according to claim 7, **characterized in that** the removed IPU unit (7) is configured to be plugged into a computer to access the data and/or operating parameters stored and/or acquired thereon.

9. The actuating unit (1) according to claim 7 or 8, **characterized in that** the removed IPU unit (7) is adapted to install necessary updates and/or new programs.

10. The actuating unit (1) according to one of claims 2 to 8, **characterized in that** the underside of the switch housing (3) is provided with a non-slip coating.

11. The actuating unit (1) according to one of claims 2 to 9, **characterized in that** a bidirectional data exchange is provided between the IPU unit (7) and a charging device during a charging process, and data exchange, in particular of all data and/or operating parameters of all application parts (14), is provided between the charging device and a cloud.

12. The actuating unit according to one of claims 2 to 10, **characterized in that** a communication connection (15) of the actuating unit with a smartphone (16) is provided, in particular for operating the respective application part (14) and for use as a display of the IPU unit (7).

13. A Surgical treatment system comprising an actuating unit (1) having the features according to one of the preceding claims and an electromotively-operated surgical instrument (2), whose motor system (6), in particular electric motor, which is configured for operation with an energy storage unit (5), is housed in an application part (14), at the proximal end (17) or end region of which a cable connection for the power supply of at least of the motor system, in particular of the electric motor, and/or for the transmission of data and/or operating parameters is formed or arranged.

## Revendications

1. Unité d'actionnement (1), en particulier unité de commutateur à pédale ou unité de commutateur à main, pour un ou d'un instrument (2) chirurgical actionné par moteur électrique, de préférence de type mini-invasif avec
un boîtier de commutateur (3) séparé de l'instrument (2) chirurgical ainsi qu'espacé spatialement qui est formé ou pourvu d'un bâti (4), de socles ou moyens de support similaires pour supporter le boîtier de commutateur (3) sur une base, de préférence un sol ou de moyens de fixation pour la fixation à une base de fixation, de préférence une table d'opération ou un bras de robot,
au moins une unité d'alimentation en énergie (5), de préférence une unité d'accumulation d'énergie qui est conçue pour une alimentation en énergie au moins d'un système de moteur (6), en particulier d'un moteur électrique, de l'instrument (2) chirurgical à raccorder à l'unité d'actionnement (1),
un appareil de commande et de surveillance (7) qui est réalisé afin d'exécuter et/ou de surveiller au moins l'une des fonctions suivantes :
la réception et/ou l'émission de données à une unité d'actionnement d'instrument (1) externe, de préférence une unité de commutateur à pédale ou une unité de commutateur à main,
l'émission et/ou la réception de paramètres de fonctionnement et de signaux de fonctionnement à/d'un instrument (2) chirurgical raccordé à celui-ci de préférence par rapport à son système de moteur et
l'enregistrement de données et/ou de paramètres de fonctionnement,
un câble (8) et/ou un raccord de câble (9) est prévu pour raccorder le câble (8) en vue du couplage électrique de l'unité d'actionnement (1) directement avec un instrument (2) chirurgical pour un fonctionnement de son système de moteur, en particulier de son moteur électrique, au moyen de l'unité d'alimentation en énergie (5) intégrée dans l'unité d'actionnement (1) et de préférence pour une transmission des données et/ou paramètres de fonctionnement, en particulier de signaux électriques et données pertinents pour la sécurité,
dans laquelle l'appareil de commande et de surveillance (7) est intégré dans le boîtier de commutateur (3) et est une unité IPU et en ce que l'unité d'alimentation en énergie (5) est intégrée dans l'unité IPU et peut alimenter en énergie l'instrument (2) chirurgical actionné par moteur électrique avec son système de moteur (6) mais aussi tous les autres composants intégrés dans le boîtier de commutateur (3) de l'appareil de commande et de surveillance (7), **caractérisée en ce qu'**une
liaison de communication de l'unité IPU avec un smartphone ou une tablette est prévue pour commander la part d'application respective et pour l'utiliser comme écran de l'unité IPU, de sorte que l'unité IPU puisse surveiller l'état de charge de l'unité d'accumulation d'énergie et/ou puisse mettre en et hors service l'apport d'énergie et/ou puisse régler le paramètre de moteur individuel.

2. Unité d'actionnement (1) selon la revendication 1, **caractérisée en ce que** l'appareil de commande et de surveillance (7) présente les composants suivants : un dispositif de capteur de signal (10) qui est réalisé de telle manière qu'il distribue les paramètres de fonctionnement et/ou les signaux de fonctionnement à son moteur électrique, un dispositif d'alimentation en courant du moteur (11) qui est réalisé de telle manière qu'il commande l'alimentation en courant du moteur, un dispositif de détection de données (12) central qui est réalisé de telle manière qu'il reçoit, enregistre, détecte et distribue les données et/ou les paramètres de fonctionnement de la partie d'application que forment le système de moteur (6) et l'instrument (2) chirurgical raccordé à celui-ci, et une unité d'émission (13) qui est réalisé de telle manière qu'elle envoie les données et/ou les paramètres de fonctionnement à un smartphone ou une tablette externe et l'unité IPU (7) qui est intégrée dans le boîtier de commutateur (3) de l'unité d'actionnement (1).

3. Unité d'actionnement (1) selon la revendication 1, **caractérisée en ce que** l'unité d'accumulation d'énergie (5) est prévue de manière centrale dans l'unité IPU (7).

4. Unité d'actionnement (1) selon l'une quelconque des revendications 1 à 3, **caractérisée en ce que** l'unité d'accumulation d'énergie (5) est un accumulateur ou une batterie.

5. Unité d'actionnement (1) selon l'une quelconque des revendications 2 à 4, **caractérisée en ce que** l'unité IPU (7) est réalisé afin de détecter de manière centralisée des données et/ou des paramètres de fonctionnement dans le boîtier de commutateur (3) de l'unité d'actionnement (1).

6. Unité d'actionnement (1) selon l'une quelconque des revendications 2 à 5, **caractérisée en ce que** l'unité IPU (7) présente une interface de données, en particulier une interface pour enficher un support de stockage, pour lire toutes les données et/ou paramètres de fonctionnement de l'historique du moteur de toutes les parties d'application (14).

7. Unité d'actionnement (1) selon l'une quelconque des revendications 2 à 6, **caractérisée en ce que** l'unité IPU (7) peut être retirée complètement du boîtier de commutateur (3), en particulier pour nettoyer et/ou préparer le boîtier de commutateur (3).

8. Unité d'actionnement (1) selon la revendication 7, **caractérisée en ce que** l'unité IPU (7) retirée est réalisée de manière à être enfichée sur un ordinateur afin de pouvoir accéder aux données et/ou paramètres de fonctionnement enregistrés dessus et/ou détectés.

9. Unité d'actionnement (1) selon la revendication 7 ou 8, **caractérisée en ce que** l'unité IPU (7) retirée est réalisée de manière à précharger/installer des mises à jour nécessaires et/ou de nouveaux programmes.

10. Unité d'actionnement (1) selon l'une quelconque des revendications 2 à 9, **caractérisée en ce que** le côté inférieur du boîtier de commutateur (3) est pourvu d'un revêtement antidérapant.

11. Unité d'actionnement (1) selon l'une quelconque des revendications 2 à 9, **caractérisée en ce qu'**un échange de données bidirectionnel est prévu entre l'unité IPU (7) et un appareil de charge pendant un processus de charge et un échange de données, en particulier de toutes les données et/ou paramètres de fonctionnement de toutes les parties d'application (14) est prévu entre l'appareil de charge et un nuage.

12. Unité d'actionnement selon l'une quelconque des revendications 2 à 11, **caractérisée en ce qu'**une liaison de communication (15) de l'unité d'actionnement avec un smartphone (16) est prévue, en particulier pour commander la partie d'application (14) respective et pour l'utiliser comme affichage de l'unité IPU (7).

13. Système de traitement chirurgical avec une unité d'actionnement (1) avec les caractéristiques selon l'une quelconque des revendications précédentes et un instrument (2) chirurgical actionné par moteur électrique, dont le système de moteur, en particulier le moteur électrique qui est conçu pour fonctionner avec une unité d'accumulation d'énergie (5), est logé dans une partie d'application (14) au niveau de l'extrémité (17) ou zone d'extrémité proximale de laquelle est réalisé ou agencé un raccord de câble pour alimenter en énergie au moins le système de moteur, en particulier le moteur électrique, et/ou pour transmettre des données et/paramètres de fonctionnement.
